Europäisches Patentamt

European Patent Office

Office européen des brevets

(19)

(11) Publication number: **0 095 751**
**B1**

(12) # EUROPEAN PATENT SPECIFICATION

(45) Date of publication of patent specification: **05.03.86**

(51) Int. Cl.⁴: **G 01 N 33/74,** A 61 K 37/24

(21) Application number: **83105277.4**

(22) Date of filing: **27.05.83**

(54) Secretin composition and method for preventing the adsorption of secretin.

(30) Priority: **28.05.82 JP 89807/82**

(43) Date of publication of application:
**07.12.83 Bulletin 83/49**

(45) Publication of the grant of the patent:
**05.03.86 Bulletin 86/10**

(84) Designated Contracting States:
**AT BE CH DE FR GB IT LI NL SE**

(56) References cited:
**EP-A-0 063 328**
**US-A-3 940 480**
**US-A-4 357 310**

(73) Proprietor: **Eisai Co., Ltd.**
**6-10, Koishikawa 4-chome Bunkyo-ku**
**Tokyo 112 (JP)**

(72) Inventor: **Kakimoto, Fumio**
**5-133-3, Mitsuike-cho Unuma**
**Kagamigahara-shi Gifu Prefecture (JP)**
Inventor: **Asakawa, Naoki**
**105-2, Nakaoida-cho**
**Kagamigahara-shi Gifu Prefecture (JP)**
Inventor: **Ishibashi, Yasuo**
**880-86, Nagaraobusa**
**Gifu Prefecture (JP)**
Inventor: **Shinoda, Aishin**
**1-292, Aza-fusoudai Oaza-takao**
**Fusou-cho Niwa-gun Aichi Prefecture (JP)**
Inventor: **Miyake, Yasuo**
**1-17, Aza-todomegi Hashizume**
**Inuyama-shi Aichi Prefecture (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Courier Press, Leamington Spa, England.

## Description

This invention relates to an adsorption-prevented secretin composition and a method for preventing the adsorption of secretin.

Secretin is a kind of gastrointestinal hormones, that is, a pancretic juice secreting hormone and is a substance useful in the treatments or diagnoses of various diseases relating to the pancreas and the gall bladder. Since this substance is a hormone, its single dosage is limited to a very small amount as little as several micrograms, and therefore this dosage must be strictly observed. In other words, there is a special requirement that this substance must be administered at exactly the same dosage level as incorporated in its dosable preparation.

On the other hand, it is well known that proteins and peptides present in an aqueous solution have general tendency to stick on a vitreous material, thereby lowering their contents to considerable extents compared with their initial contents in dosable preparations. When insulin is placed in an infusion container for example, a considerable portion of the insulin is instantly adsorbed on the glass surface of the container, and this adsorption has the tendency to take place more easily where the concentration of insulin is low [YAKUZAIGAKU, 39, 107—111 (1979)]. More specifically, a peptide hormone such as insulin is generally in a state ready to undergo adsorption on a glass container since its dosage is intrinsically restricted to a very small amount and its percentage loss due to the adsorption is great as its original content is low, resulting in a considerably-lowered dosage. This loss, induced by the adsorption of the peptide hormone on the glass container, is a phenomenon equally observed with respect to secretin to which the present invention relates [Chem. Pharm. Bull., 27(12), 3160—3163, (1979)]. In other words, secretin is a peptide hormone, the dosage of which is also very small like insulin, and therefore, when it is present in an aqueous solution, it is readily adsorbed on a glass container and its percentage loss due to the adsorption becomes considerably high. For example, even if secretin is precisely taken to a desired amount in a container while carefully avoiding its adsorption to the container and others upon forming it into a dosable preparation, a considerable portion thereof is adsorbed on the glass wall of a syringe or infusion container when it is transferred to a syringe or placed in an infusion container for its infusion as a mixture together with a solution of infusion upon actual administration, resulting in the occurrence of serious influence. With the foregoing in view, the present inventors carried out various investigations with a view toward developing a technical solution capable of preventing the adsorption of secretin, which is present in an aqueous solution, on its container. As a result, it was surprisingly found that the above object may be achieved by incorporating a basic amino acid in the aqueous solution of secretin, resulting in a patent application to which European Patent Application EP—A—63328 has been allotted.

In the meantime, plastic syringe barrels (made of polypropylene), plastic infusion bottles (made of polypropylene or polyethylene), plastic-made dripping guide tubes (made of polyvinyl chloride), etc. have found wide-spread commercial utility, in addition to their corresponding glass-made products. Under the circumstances, there is an increased opportunity for secretin to be adsorbed. With such current circumstances in view, the present inventors have expanded their research work with a view toward developing a technical solution which may be effective in avoiding adsorption of secretin even when secretin is brought into contact with the plastic walls of various containers. As a result, it has been found that the above object can be attained by an addition of one or more substances selected from the group consisting of lecithin, ethylene oxide-propylene oxide copolymers, hydroxypropylcellulose, methylcellulose, polyoxyethylene hydrogenated castor oil, polyethylene glycol sorbitan oleate and methylcyclodextrin to an aqueous solution of secretin, leading to completion of this invention.

An object of this invention is to provide a secretin-containing composition in which secretin is prevented from being adsorbed on the wall of a container.

Another object of this invention is to provide a method for preventing secretin from being adsorbed on the wall of a container.

In one aspect of this invention, there is thus provided a secretin-containing composition which comprises, as an intimate mixture or a combination of separate units, secretin and one or more additives selected from the group consisting of lecithin, ethylene oxide-propylene oxide copolymers, hydroxypropylcellulose, methylcellulose, polyoxyethylene hydrogenated castor oil, polyethylene glycol sorbitan oleate and methylcyclodextrin.

In another aspect of this invention, there is also provided a method for preventing the adsorption of secretin, which method comprises incorporating one or more additives selected from the group consisting of lecithin, ethylene oxide-propylene oxide copolymers, hydroxypropylcellulose, methylcellulose, polyoxyethylene hydrogenated castor oil, polyethylene glycol sorbitan oleate and methylcylodextrin, in a secretin-containing aqueous solution.

Use of such additives has been found to be very effective in preventing secretin from being adsorbed on the walls of plastic- or glass-made syringe barrels, infusion bottles or dripping guide tubes, thereby permitting administration of secretin at accurate dosage levels.

The above and other objects, features and advantages of the present invention will become apparent from the following description and the appended claims, taken in conjunction with the accompanying drawings.

In the accompanying drawings:

Fig. 1 is a histogram showing the results of Experiment 1, namely, the effects of types and concentations of additives according to this invention on percentage recoveries of secretin when the secretin was brought into contact with a polyethylene-made test tube;

Fig. 2 is a histogram showing some results of Experiment 2, namely, the effects of types and concentrations of additives according to this invention and times of contact on percentage recoveries of secretin when the secretin was brought into contact with a polyethylene-made test tube or a polypropylene-made syringe barrel;

Fig. 3 is a histogram showing certain results of Experiment 2, namely, the effects of types and concentrations of additives according to this invention and times of contact on percentage recoveries of secretin when the secretin was brought into contact with a polyethylene-made test tube or a glass-made syringe barrel; and

Fig. 4 is a histogram showing the results of Example 3, namely, the effects of concentrations of additives according to this invention and times of contact on percentage recoveries of secretin when the secretin was brought into contact with a PVC-made tube.

In the present invention, any secretin may be used regardless of its preparation route. However, the present invention may generally be practiced using secretin which has been obtained by extracting crude secretin from the small intestine mucous membranes of mammals, especially cattle, pigs, etc., and then isolating and purifying same in accordance with various methods. While the dosage of secretin may vary depending on the purpose of each diagnosis or treatment, preparations containing, for example, 2000—5000 Crick Haper Raber units of secretin per ml are widely employed. However, the present invention is not restricted by the content of secretin.

Next, the additive used for prevention of the adsorption of secretin in accordance with this invention may be, as recited in Experiments which will appear later in this specification, one or more substances selected from the group consisting of lecithin, ethylene oxide-propylene oxide copolymers, hydroxypropylcellulose, methylcellulose, polyoxyethylene hydrogenated castor oil, polyethylene glycol sorbitan oleate and methylcyclodextrin. The effectiveness of these substances was unexpectedly found out as a result of an experiment in which a number of substances was investigated as to their possible adsorption-preventive effects for secretin. Therefore, there is no generic concept specifically embracing these substances only and the mechanism which leads to the prevention of adsorption has not been elucidated. However, the present inventors have uncovered that these substances can be specifically chosen for prevention of the adsorption of secretin and these substances can exhibit practically perfect adsorption-preventive effects at significantly low concentrations. It is here that the novelty and unobviousness of this invention can be recognized. It is desirous that one or more additives selected from the group of these substances are added in such a total amount that, when a secretin-containing composition is formed into an aqueous solution, the total content of the additives in the solution is 0.001% or higher. It is also desirous that the contents of such additives are so low that they do not by themselves exhibit any pharmacological effects when administered in living bodies. In this sense, it is preferred to limit the total concentration of one or more additives below 1% in each aqueous solution of secretin. It should however be borne in mind that the present invention is not necessarily limited to the concentration range. It is clear that the adsorption-preventive effects of one or more additives according to this invention have been brought about as a result of their effects to the contacting surface of a container because similar effects can also be obtained even if the additives are added later to each aqueous secretin solution, although the mechanism in which one or more additives according to this invention eventually exhibit the adsorption-preventive effects is not clear as mentioned above. Thus, the minimum total amount of one or more additives according to this invention, which amount is required to draw out the effectiveness of the additives, is not governed by the content of secretin but is determined in accordance with the surface area of the inner wall of a container. Thus, if the maximum surface area of a container which is brought into contact with an aqueous secretin solution during the use of the solution is known, it is possible to determine the total amount of one or more additives according to this invention to be added to each secretin-containing composition according to this invention. However, it is impossible to have a correct figure as to the surface area from the beginning, it is imposible to determine accurately the total amount of one or more additives to be incorporated in the composition because the surface area cannot be found from the beginning. However, as indicated by the Experiments to be given later in this specification, it is suitable for achieving the objects of this invention when the one or more additives are present within the above-described concentration range when the secretin-containing composition is formed into an aqueous solution, namely at a total concentration of 0.001%—1%.

The composition of this invention may be present in several types of pharmaceutical preparation forms. Namely, it is not always necessary to provide both secretin and one or more additives according to this invention as an intimate mixture in the same composition from the beginning. It is also feasible to provide them as a combination of separate units so that they may be mixed together prior to using them. Thus, the composition according to this invention may take, as specific examples, preparation forms as given below:

(i) An aqueous composition obtained by causing secretin and one or more additives according to this invention to be present as an intimate mixture in the same aqueous medium;

(ii) A composition containing secretin and one or more additives according to this invention as separate aqueous solutions so that they may be mixed together on use;

(iii) A composition obtained by causing secretin and one or more additives according to this invention to be present as an intimate mixture of lyophilized powders, to which a separately-furnished solvent is added on use;

(iv) A composition containing secretin and one or more additives according to this invention as separate lyophilized powders, which may be converted into their corresponding aqueous solutions and then mixed together on use; and (v) A composition containing secretin as lyophilized powder and one or more additives according to this invention as an aqueous solution, which may be combined together on use.

Preparation of the above-mentioned various aqueous solutions and lyophilized powders may be readily carried out by methods commonly employed in the art. It is also free in the present invention to add a suitable stabilizer, buffer, etc. to the aqueous solutions or to incorporate a buffer in the lyophilized powders or an appropriate excipient or the like for lyophilization.

This invention also provides a method for preventing secretin in a secretin-containing aqueous solution from being adsorbed on the wall of a container, which aqueous solution is in contact with the wall of the container. Namely, a separately-furnished aqueous solution of one or more of the additives according to this invention may be added to a secretin-containing infusing solution or secretin-containing ampule solution, or secretin may be incorporated in an already-provided infusing solution or ampule solution which contains one or more of the additives according to this invention in order to prevent the adsorption of secretin on the contacting wall of a container. Needless to say, these methods belong to the same technical concept as the above-described invention pertaining to adsorption-prevented compositions.

The effects of this invention will be described in the following Experiments:

Experiment 1:
(Samples)

Each of the additives, methylcellulose, polyoxyethylene hydrogenated castor oil, lecithin, hydroxypropylcellulose, an ethylene oxide-propylene oxide copolymer, polyethylene glycol sorbitan oleate and methylcyclodextrin, was added to a 0.9% aqueous NaCl solution at the concentration levels of 0.1%, 0.01%, 0.001%, 0.0001% and 0.00001% respectively, thereby providing test samples.

Besides, 0.9% aqueous NaCl solutions containing bovine serum albumin respectively at the various concentration levels as well as a 0.9% aqueous NaCl solution were provided as controls (i.e., reference samples).

(Procedure)

To a polyethylene-made test tube containing 50 Crick Haper Raber units of secretin which was in a lyophiized state, 2 ml of each of the samples was added. Upon an elapsed time of 10 minutes, 100 µl of the resultant solution was drawn in a microsyringe and charged in a high-speed liquid chromatography column. The amount of non-adsorbed secretin was measured by high-speed liquid chromatography and the percentage recovery of secretin was determined.

By the way, the high-speed liquid chromatography was operated at the wavelength of 210 nm, using $CH_3CN$—$H_2O$—70% $HClO_4$ (40:60:0.5) as its moving phase. These conditions for the high-speed liquid chromatography were also used in the subsequent Experiments.

(Results)

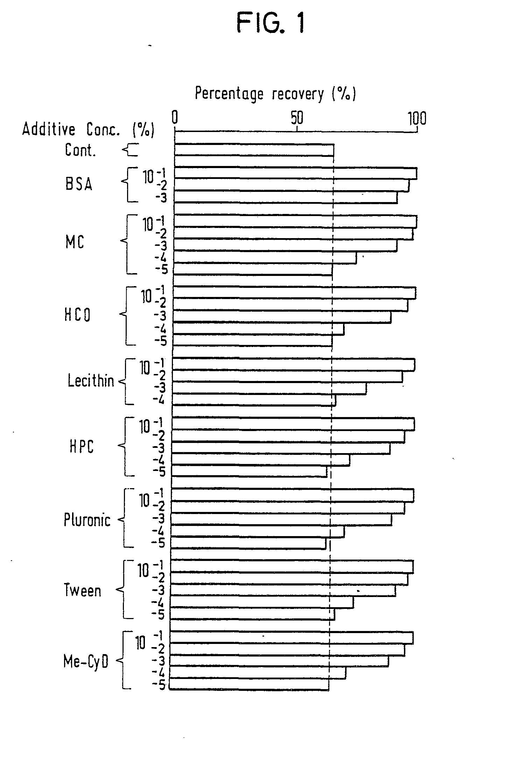

Results are illustrated in Fig. 1 In the histogram, are shown the percentage recoveries when bovine serum albumin (BSA), methylcellulose(MC), polyoxyethylene hydrogenated castor oil(HCO), lecithin(Lecithin), hydroxypropylcellulose(HPC), the ethylene oxide-propylene oxide copolymer(Pluronic®), polyethylene glycol sorbitan oleate(Tween®) and methylcyclodextrin(Me-Cyd®) were added as additives to the 0.9% aqueous NaCl solution respectively. Furthermore, Cont. stands for the 0.9% aqueous NaCl solution without any additives.

On the other hand, $10^{-1}$, $10^{-2}$, $10^{-3}$, $10^{-4}$, and $10^{-5}$ indicate the percentage recoveries of secretin when their corresponding additives were contained respectively at the given concentration levels, i.e., at 0.1%, 0.01%, 0.001%, 0.0001% and 0.00001% respectively.

Bovine serum albumin is a conventionally-known adsorption-preventive agent. It has been known that an inclusion of bovine serum albumin at 0.1% provides the full percentage recovery, i.e., 100%. However, use of bovine serum albumin for the purpose of preventing the adsorption of secretin involves some problems from the practical viewpoint.

It will be appreciated from Fig. 1 that the additives according to this invention can each serve as an adsorption-preventive agent comparable with bovine serum albumin. Moreover, it is also understood that the additives according to this invention can each exhibit an effect of preventing secretin from being adsorbed on a container when it is added at $10^{-3}$%, namely, 0.001% or more.

Experiment 2:
(Samples)

Each of the additives, the ethylene oxide-propylene oxide copolymer and polyoxyethylene hydrogenated castor oil, was added to a 0.9% aqueous NaCl solution respectively at 0.001%, 0.01% and 0.1%, thereby providing test samples. Besides, a 0.9% aqueous NaCl solution was also furnished as a control.

(Procedure)

To two polyethylene-made test tubes each containing 50 Crick Haper Raber units of secretin which was in a lyophilized state, 2 ml of each of the samples was individually added to dissolve secretin. The resultant solution was immediately taken in a 5-ml glass-made syringe barrel and a 5-ml polypropylene-made syringe barrel respectively. After an elapsed time of each of 1, 5 and 10 minutes, portions of the solutions were transferred to spit bowls, each followed by an addition of a mixed solvent of 70% $HClO_4$—$CH_3CN$ (1:9). The amounts of non-adsorbed secretin were measured by the high-speed liquid chromatography and the percentage recoveries were then determined.

(Results)

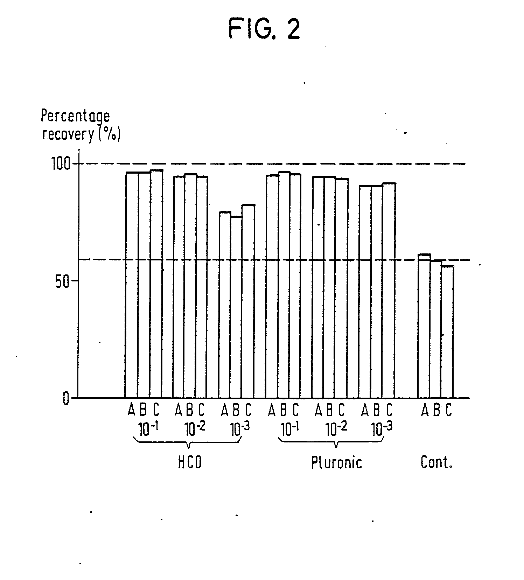

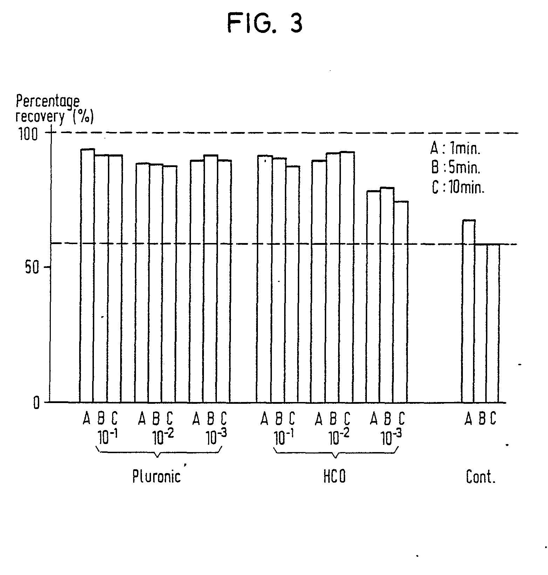

Results are shown in Figs. 2 and 3. In each of the histograms, Cont., Pluronic® and HCO indicate respectively the percentage recoveries obtained when the 0.9% aqueous NaCl solution was used without any additives and the ethylene oxide-propylene oxide copolymer and polyoxyethylene hydrogenated castor oil were added to 0.9% aqueous NaCl solutions. On the other hand, $10^{-1}$, $10^{-2}$ and $10^{-3}$ indicate the percentage recoveries of secretin when their corresponding additives were contained respectively at the given concentration levels, i.e., at 0.1%, 0.01% and 0.001% respectively. "A", "B" and "C" mean respectively the percentage recoveries obtained after passage of the respective times of contact, i.e., 1 minute, 5 minutes and 10 minutes. The histogram of Fig. 2 show test results where the solutions were taken in the polypropylene-made syringe barrels whereas the histogram of Fig. 3 illustrate test results where the solutions were taken in the glass-made syringe barrels.

From Figs. 2 and 3, it is appreciated that the above additives according to this invention have effects to prevent adsorption of secretin which is observed when secretin is brought into contact with the walls of polyethylene-made test tubes, or polypropylene- or glass-made syringe barrels.

Experiment 3:
(Samples)

The ethylene oxide-propylene oxide copolymer was added to 0.9% aqueous NaCl solutions respectively at 0.001% and 0.01% thereby providing test samples. Besides, a 0.9% aqueous NaCl solution was furnished as control.

(Procedure)

To polyethylene-made test tubes each containing 50 Crick Haper Raber units of secretin which was in a lyophilized state, the samples were respectively added to the volumes of 2 ml to form solutions. Here, the amounts of non-adsorbed secretin were measured by the high-speed liquid chromatography. Thereafter, the solutions were transferred respectively in 30 cm-long, PVC-made tubes of infusion sets. The amounts of non-adsorbed secretin were periodically measured by the high-speed liquid chromatography and the percentage recoveries were determined relative to the amounts of secretin before the transference of the solutions to their respective PVC-made tubes.

(Results)

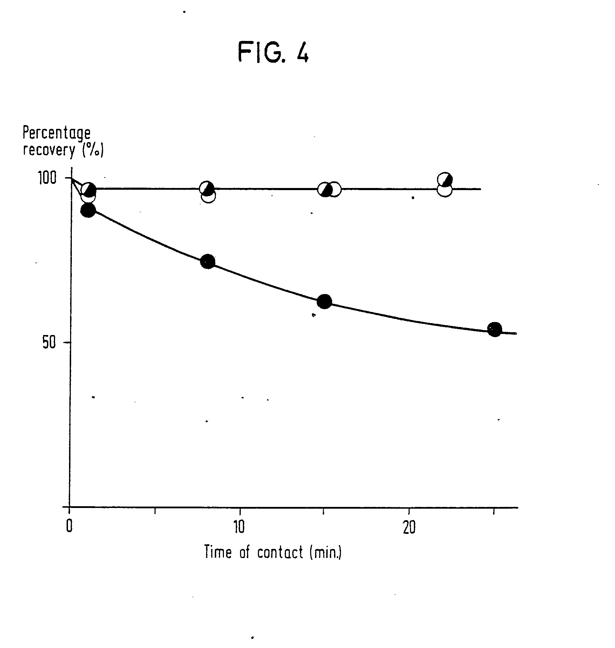

Results are shown in Fig. 4, in which Line(—●—) indicates the percentage residue of secretin where the aqueous NaCl solution was used without any additive, Line(—◕—) indicates the percentage residue of secretin where the ethylene oxide-propylene oxide copolymer was added to the 0.9% aqueous NaCl solution at 0.01%, and Line (—○—) represents the percentage residue of secretin where the ethylene oxide-propylene oxide copolymer was added to the 0.9% aqueous NaCl solution at 0.0001%. From Fig. 4, it is envisaged that the above additive according to this invention has effects to prevent adsorption of secretin which is observed when secretin is brought into contact with the wall of the PVC-made tube of an infusion set.

The present invention will next be described specifically in the following Examples.

Example 1:

An aqueous solution containing 0.05 g of polyoxyethylene hydrogenated castor oil, 0.8 g of sodium chloride and 5000 Crick Haper Raber units of secretin in 100 ml of a 0.03-M citrate/phosphate buffer of pH 4.0 was aseptically prepared. It was filled in 1-ml portions in ampules and then hermetically sealed.

Example 2:

An aqueous solution containing 0.02 g of polyethylene glycol sorbitan oleate, 0.03 g of the ethylene oxide-propylene oxide copolymer, 0.8 g of sodium chloride and 5000 Crick Haper Raber units of secretin in 100 ml of a 0.03-M citrate/phosphate buffer of pH 4.0 was aseptically prepared. It was filled in 1-ml portions in ampules and then hermetically sealed.

Example 3:

An aqueous solution containing 2 g of L-alanine, 0.05 g of lecithin and 5000 Crick Haper Raber units of secretin in 50 ml of a 0.03-M citrate/phosphate buffer of pH 4.5 was aseptically prepared. It was filled in 0.5-ml portions in ampules, lyophilized and then hermetically sealed. On the side, ampules each containing 1 ml of distilled injection water were prepared as ampules containing a dissolving solution.

Example 4:

An aqueous solution containing 2 g of L-alanine, 0.2 g of L-cystein and 10000 Crick Haper Raber units in 50 ml of a 0.03-M citrate/phosphate buffer of pH 4.5 was aseptically prepared. It was filled in 0.5-ml portions in vials, lyophilized and then hermetically sealed. On the side, a 0.1% aqueous solution of methylcellulose was aseptically prepared, filled in 1-ml portions in ampules and then hermetically sealed, thereby providing ampules containing a dissolving solution.

**Example 5:**

Following the procedure of Example 4, vials containing lyophilized secretin were prepared. On the side, a 0.1% aqueous solution of hydroxypropylcellulose was aseptically prepared, filled in 1-ml portions in ampules and then hermetically sealed. thereby providing ampules containing a dissolving solution.

**Example 6:**

An aqueous solution containing 2 g of L-alanine, 0.05 g of methylcyclodextrin and 5000 Crick Haper Raber units of secretin in 50 ml of a 0.03-M citrate/phosphate buffer of pH 4.5 was aseptically prepared, filled in 0.5-ml portions in ampules, lyophilized and then hermetically sealed. On the side, ampules each containing 1 ml of distilled injection water were provided as ampules containing a dissolving solution.

**Example 7:**

An aqueous solution containing 2 g of L-alanine, 0.05 g of lecithin and 5000 Crick Haper Raber units of secretin in 50 ml of a 0.03-M citrate/phosphate buffer of pH 4.5 was aseptically prepared, filled in 0.5-ml portions in vials, lyophilized and then hermetically sealed. On the side, a 0.1% aqueous solution of polyoxyethylene hydrogenated castor oil was aseptically prepared, filled in 1-ml portions in ampules and hermetically sealed, thereby providing ampules containing a dissolving solution.

**Example 8:**

A 1% aqueous solution of polyoxyethylene hydrogenated castor oil was aseptically prepared, filled in 2-ml portions in ampules, and hermetically sealed, thereby providing ampules containing a solution effective in preventing the adsorption of secretin.

**Example 9:**

An aqueous solution containing 0.5% of the ethylene oxide-propylene oxide copolymer and 0.5% of polyethylene glycol sorbitan oleate was aseptically prepared, filled in 2-ml portions in ampules and then hermetically sealed, thereby providing ampules containing a solution effective in preventing the adsorption of secretin.

**Claims**

1. A secretin - containing composition characterised in that it comprises an intimate mixture or a combination of separate units, secretin and one or more additives selected from the group consisting of lecithin, ethylene oxide-propylene oxide copolymers, hydroxypropylcellulose, methylcellulose, polyoxyethylene hydrogenated castor oil, polyethylene glycol sorbitan oleate and methylcyclodextrin.

2. The secretin-containing composition according to Claim 1, wherein the one or more additives are present in such a total amount that, when the secretin-containing composition is converted into an aqueous solution, the total concentration of the additives in the aqueous solution falls within the range of 0.001—1%.

3. The secretin-containing composition according to Claim 1, wherein the composition is in the form of an aqueous solution.

4. The secretin-containing composition according to Claim 2, wherein the composition is in the form of an aqueous solution.

5. The secretin-containing composition according to Claim 1, wherein the composition is in a lyophilized powder form.

6. The secretin-containing composition according to Claim 2, wherein the composition is in a lyophilized powder form.

7. The secretin-containing composition according to Clain 1, wherein the composition is formed of a combination of lyophilized powder and an aqueous solution.

8. The secretin-containing composition according to Claim 2, wherein the composition is formed of a combination of lyophilized powder and an aqueous solution.

9. A method for preventing the adsorption of secretin, which method comprises incorporating one or more additives selected from the group consisting of lecithin, ethylene oxide-propylene oxide copolymers, hydroxyproplycellulose, methylcellulose, polyoxyethylene hydrogenated castor oil, polyethylene glycol sorbitan oleate and methylcyclodextrin, in a secretin-containing aqueous solution.

10. The method for preventing the adsorption of secretin according to Claim 9, wherein the one or more additives are incorporated in such an amount that their total concentration in the secretin-containing aqueous solution falls within the range of 0.001—1%

**Patentansprüche**

1. Secretin enthaltende Zusammsetzung, dadurch gekennzeichnet, dass sie ein inniges Gemisch oder eine Kombination getrennter Einheiten von Secretin und einem oder mehreren Hilfsstoffen, ausgewählt aus der Gruppe, bestehend aus Lecithin, Ethylenoxid - Propylenoxid - Copolymeren, Hydroxypropylcellulose, Methylcellulose, Polyoxyethylen - hydriertes Rizinusöl, Polyethylen - glykol - sorbitanoleat und Methylcyclodextrin, umfasst.

2. Secretin enthaltende Zusammensetzung nach Anspruch 1, wobei einer oder mehrere Hilfsstoffe in einer Gesamtmenge vorliegen, dass, wenn die Secretin enthaltende Zusammensetzung in eine wässrige Lösung überführt wird, die Gesamtkonzentration der Hilfsstoffe in der wässrigen Lösung in dem Bereich von 0,001 bis 1% liegt.

3. Secretin enthaltende Zusammensetzung nach Anspruch 1, wobei die Zusammensetzung in Form einer wässrigen Lösung vorliegt.

4. Secretin enthaltende Zusammensetzung nach Anspruch 2, wobein die Zusammensetzung in Form einer wässrigen Lösung vorliegt.

5. Secretin enthaltende Zusammensetzung nach Anspruch 1, wobei die Zusammensetzung in Form eines lyophilisierten Pulvers vorliegt.

6. Secretin enthaltende Zusammensetzung nach Anspruch 2, wobei die Zusammensetzung in Form eines lyophilisierten Pulvers vorliegt.

7. Secretin enthaltende Zusammensetzung nach Anspruch 1, wobei die Zusammensetzung eine Kombination aus einem lyophilisierten Pulver und einer wässrigen Lösung darstellt.

8. Secretin enthaltende Zusammensetzung nach Anspruch 2, wobei die Zusammensetzung eine Kombination aus einem lyophilisierten Pulver und einer wässrigen Lösung darstellt.

9. Verfahren zur Verhinderung der Adsorption vin Secretin, gekennzeichnet durch Inkorporieren eines oder mehrerer Hilfsstoffe, ausgewählt aus der Gruppe, bestehend aus Lecithin, Ethylenoxid - Propylenoxid - Copolymeren, Hydroxypropylcellulose, Methylcellulose, Polyoxyethylenhydriertes Rizinusöl, Polyethylenglykol - sorbitanoleat und Methylcyclodextrin, in eine Secretin enthaltende wässrige Lösung.

10. Verfahren zur Verhinderung der Adsorption von Secretin nach Anspruch 9, wobei der bzw. die genannten Hilfsstoffe in einer Menge inkorporiert werden, dass ihre Gesamtkonzentration in der Secretin enthaltenden wässrigen Lösung in dem Bereich von 0,001 bis 1% leigt.

## Revendications

1. Composition contenant de la sécrétine, caractérisée en ce qu'elle comprend un mélange intime, ou une association d'unités séparées, de sécrétine et d'un ou plusieurs additifs choisis dans le groupe consistant en lécithine, copolymères oxyde d'éthylène-oxyde de propylène, hydroxypropylcellulose, méthylcellulose, polyoxyéthylène(huile de ricin hydrogénée), oléate de polyethylène - glycol - sorbitanne et méthylcyclodextrine.

2. Composition contenant de la sécrétine selon la revendication 1, dans laquelle le ou les additifs sont présents en une quantité totale telle que lorsque la composition contenant la sécrétine est transformée en une solution aqueuse, la concentration totale des additifs dans la solution aqueuse tombe dans la plage de 0,001 à 1%.

3. Composition contenant de la sécrétine selon la revendication 1, dans laquelle la composition est sous la forme d'une solution aqueuse.

4. Composition contenant de la sécrétine selon la revendication 2, dans laquelle la composition est sous la forme d'une solution aqueuse.

5. Composition contenant de la sécrétine selon la revendication 1, dans laquelle la composition est sous forme de poudre lyophilisée.

6. Composition contenant de la sécrétine selon la revendication 2, dans laquelle la composition est sous forme d'une poudre lyophilisée.

7. Composition contenant de la sécrétine selon la revendication 1, dans laquelle la composition est formée d'une association de poudre lyophilisée et d'une solution aqueuse.

8. Composition contenant de la sécrétine selon la revendication 2, dans laquelle la composition est formée d'une association de poudre lyophilisée et d'une solution aqueuse.

9. Procédé pour empêcher l'adsorption de sécrétine, lequel procédé consiste à incorporer un ou plusieurs additifs choisis dans le groupe consistant en lécithine, copolymères oxyde d'éthylène-oxyde de propylène, hydroxypropylcellulose, méthylcellulose, polyoxyéthylène - (huile de ricin hydrogénée), oléate de polyéthylène - glycol - sorbitanne et méthylcyclodextrine, dans une solution aqueuse contenant de la sécrétine.

10. Procédé pour empêcher l'adsorption de sécrétine selon la revendication 9, lequel le ou les additifs sont incorporés en quantité telle que leur concentration totale dans la solution aqueuse contenant la sécrétine tombe dans la plage de 0,001—1%.

# FIG. 1

# FIG. 2

Percentage recovery (%)

# FIG. 3

FIG. 4

Percentage recovery (%)

Time of contact (min.)